# EUROPÄISCHE PATENTANMELDUNG

(11) **EP 0 768 293 A1**
(43) Veröffentlichungstag der Anmeldung: **16.04.1997**
(21) Anmeldenummer: 96115712.0
(22) Anmeldetag: 01.10.1996
(51) Int. Cl.: C07C 45/58, C07C 47/02

(54) **Verfahren zur Herstellung von Propanal**

(30) Priorität: 13.10.1995 DE 19538206
(71) Anmelder: EC ERDÖLCHEMIE GMBH, D-50769 Köln (DE)
(72) Erfinder: Bister, Hans-Juergen, Dr., 40479 Düsseldorf (DE); Schmitz, Josef, Dr., 50181 Bedburg (DE); Stüwe, Arnd, Dr., 51373 Leverkusen (DE)
(74) Vertreter: Zobel, Manfred, Dr.

(57) **Zusammenfassung**

Propanal kann durch Isomerisierung von Propylenoxid bei 150 bis 400°C an einem sauren SiO₂-haltigen Katalysator hergestellt werden. Vorteilhaft ist ein H₃PO₄-beladenes SiO₂, bei dem der H₃PO₄-Gehalt 10-65 % des Gesamtgewichts von H₃PO₄ und SiO₂ ausmacht.

## Beschreibung

Die vorliegende Erfindung betrifft ein Verfahren zur Herstellung von Propanal durch katalytische Isomerisierung von Propylenoxid bei erhöhter Temperatur in der Gasphase.

Aus DE-AS 10 35 635 (und den darin zitierten DE-PS 528 822 und 535 651) ist es bekannt, aus α-Olefinoxiden, wie Propylenoxid, in der Gasphase in Gegenwart von Katalysatoren und bei erhöhter Temperatur die entsprechenden isomeren Aldehyde, wie Propanal, herzustellen. Als Katalysatoren werden Substanzen, wie Al₂O₃, Silicagel, Kieselgur, Aluminiumborat, Alaune, Li₃PO₄, Mg₂P₂O₇, Oxide der Übergangsmetalle, ZnCl₂, PbCl₂, CdCl₂, empfohlen. Im Ausführungsbeispiel dieser DE-AS wird zur Isomerisierung von Propylenoxid stückiges Al₂O₃ eingesetzt. Eine neuere Untersuchung (Neftekhimiya 13 (1973), 401-406; zitiert nach C.A. 79, 77 995) zeigt jedoch, daß mit einem der in der obigen DE-AS genannten Katalysatoren, nämlich mit Li₃PO₄ bei 230 bis 340°C Propylenoxid zu Allylalkohol isomerisiert wird. Eine weitere Literaturstelle (DD 240 742; zitiert nach C.A. 107, 79 955) beschreibt die Herstellung von 1-Propanol aus Propylenoxid, wobei in einem ersten Schritt eine Isomerisierung in Gegenwart von Ni₂P₂O₇ bei etwa 200 bis 450°C vorgenommen wird und das nicht näher beschriebene Isomerisierungsprodukt danach zu 1-Propanol hydriert wird. Als Nebenprodukte der Isomerisierung werden in den genannten Literaturstellen neben Propanal vor allem Allylalkohol, Aceton und Acrolein genannt, die ebenfalls wie das Propylenoxid 3 C-Atome enthalten, daneben aber auch Dimerisierungsprodukte mit 6 C-Atomen.

Aus EP 262 532 ist eine Isomerisierung von Alkylenoxiden an Zeolithen bei 150 bis 400°C zu einem Gemisch der oben genannten Produkte bekannt.

Die oben aufgeführten Literaturstellen zeigen, daß bei der Isomerisierung von Propylenoxid an einer Reihe von Katalysatoren Propanal entsteht, das dies aber nur bei der Verwendung von Al₂O₃ gemäß DE-AS 10 35 635 das Hauptprodukt ist, während an anderen Katalysatoren Allylalkohol als Hauptprodukt entsteht oder ein nicht näher gekennzeichnetes Produktgemisch, welches sich zu 1-Propanol hydrieren läßt.

Die Darstellung von Propanal besteht bis jetzt nur in der Isolierung dieses Stoffes aus Abfallprodukten, beispielsweise bei der Propylenoxidsynthese aus Propen oder bei der Herstellung von Allylalkohol oder Aceton aus Propylenoxid. Daneben kann Propanal durch Hydroformylierung des relativ teuren Ethylens hergestellt werden.

Es wurde nun gefunden, daß hohe Umsätze des zu isomerisierenden Propylenoxids und hohe Selektivitäten zu Propanal erreicht werden, wenn als Katalysator ein saures SiO₂-haltiges Material eingesetzt wird.

Die Erfindung betrifft demnach ein Verfahren zur Herstellung von Propanal, das dadurch gekennzeichnet ist, daß Propylenoxid bei 150 bis 400°C an einem sauren SiO₂-haltigen Material in der Gasphase isomerisiert wird.

Das erfindungsgemäß einzusetzende Propylenoxid kann nach jedem der bekanntgewordenen Herstellungsverfahren erzeugt worden sein, beispielsweise nach dem Chlorhydrin-Verfahren oder der katalytischen Umsetzung von Propylen mit beispielsweise t-Butylhydroperoxid oder Ethylbenzol-hydroperoxid. Die Reinheit von industriell erzeugtem Propylenoxid ist für das erfindungsgemäße Verfahren ausreichend.

Als Katalysator wird ein saures SiO₂-haltiges Material eingesetzt. Dies kann beispielsweise ein mit H₃PO₄ beladenes SiO₂ sein. Als SiO₂ sind hierbei alle auf dem Markt erhältlichen Typen und Qualitäten verwendbar. In bevorzugter Form wird SiO₂ als Kieselgel eingesetzt. Solches SiO₂ wird mit starken Säuren, beispielsweise mit H₃PO₄ behandelt bis der Gehalt an starken Säuren, beispielsweise an H₃PO₄ 10 bis 65 %, bevorzugt 15 bis 45 % des Gesamtgewichts von H₃PO₄ und SiO₂, ausmacht. H₃PO₄-beladenes SiO₂ wurde bisher erfolgreich zur Hydratisierung von Ethylen unter Bildung von Ethanol eingesetzt. Weitere mögliche Katalysatoren sind Schichtsilikate, die zusätzlich mit starken Säuren, beispielsweise mit H₃PO₄ belegt worden sind. Als Schichtsilikat sei beispielsweise Montmorillonit (natürlich vorkommendes Na-, K-, Mg-, Ca- und H₂O-haltiges Schichtsilikat) genannt, das beispielsweise folgende Eigenschaften nach der Säurebehandlung hat: Acidität 14-18 mg KOH/g gegen Phenolphthalein; pH-Wert einer 25 %igen Suspension in H₂O: 3,0-3,5; Schüttdichte ca. 0,75 g/cm³; BET-Oberfläche 320-400 m²/g.

Solche Katalysatoren können in stückiger Form als Festbettkatalysator oder in pulvriger Form in der Wirbelschicht angeordnet sein. In bevorzugter Weise wird H₃PO₄-beladenes SiO₂ eingesetzt.

Die Reaktionstemperatur beträgt 150 bis 400°C, bevorzugt 170 bis 320°C.

Der Reaktionsdruck ist nicht weiter kritisch, so daß bei Normaldruck, bei vermindertem Druck oder bei erhöhtem Druck gearbeitet werden kann. Der allgemeine Druckbereich beträgt 0,3 bis 30 bar, bevorzugt 0,8 bis 5 bar, besonders bevorzugt aus wirtschaftlichen Gründen Normaldruck.

Das Propylenoxid wird als solches oder verdünnt mit Inertgasen über den Katalysator geleitet. Als Inertgase kommen Stickstoff, Kohlenmonoxid, Kohlendioxid, Edelgase, C₁-C₄-Kohlenwasserstoffe oder auch das Reaktionsprodukt Propanal (Verdünnung durch Kreislauffahrweise) in Frage; in bevorzugter Weise wird Stickstoff als Verdünnungsmittel eingesetzt. Der Verdünnungsgrad mit dem Inertgas kann im weiten Bereich schwanken, beispielsweise im Bereich Propylenoxid:Inertgas = 1:0,1 bis 5, bevorzugt 1:1-3.

Als Strömungsgeschwindigkeit (LHSV = Liquid Hourly Space Velocity) wird ein Wert von 0,01 bis 2, bevorzugt von 0,1 bis 1, bezogen auf Propylenoxid, eingestellt (Liter Propylenoxid pro Liter Katalysator pro Stunde).

Das erfindungsgemäße Verfahren kann während langer Betriebszeiten von mehr als 1500 Stunden ohne einen merklichen Abfall der katalytischen Aktivität durchgeführt werden. Zeigt der Katalysator jedoch Ermüdungserscheinungen ist eine einfache Regenerierung mit Luft möglich.

Zur Durchführung des Verfahrens wird der Katalysator als Festbett oder als Wirbelbett in einem beheizbaren, im allgemeinen senkrecht stehenden Reaktionsrohr angeordnet und von oben oder von unten, im Falle der Wirbelschichtanordnung nur von unten, von Propylenoxid, bevorzugt im Gemisch mit einem Inertgas, angefahren. Grundsätzlich ist aber auch die waagerechte Anordnung des Reaktors möglich. Katalysator und Propylenoxid(-Inertgas)-Gemisch werden vorher auf die gewünschte Reaktionstemperatur gebracht. Das entnommene Reaktionsgas kann unmittelbar einer destillativen Aufarbeitung auf Propanal und Nebenprodukte zugeführt werden. Es kann jedoch auch in zwei Teile aufgeteilt werden, von denen ein Teil (30 bis 90 % des gesamten Reaktionsgases) der destillativen Aufarbeitung zugeführt wird und der andere Teil gemeinsam mit frischem Propylenoxid sowie gegebenenfalls Inertgas erneut in die katalytische Isomerisierung zurückgeführt wird. Bei der destillativen Aufarbeitung kann man als Kopfprodukt das gewünschte Propanal sowie nicht kondensierbare gasförmige Anteile des Reaktionsgasgemisches und als Sumpfprodukt Nebenprodukte, wie Aceton, Allylalkohol und höhersiedende Anteile erhalten,

Das erfindungsgemäße Verfahren zeichnet sich durch einen hohen Umsatz des Propylenoxids und eine hohe Selektivität zum gewünschten Propanal aus.

### Beispiele

### Katalysator-Herstellung

1 Gew.-Teil SiO₂ (Fa. Grace) wurde mit 1 Gew.-Teil 50 %iger H₃PO₄ versetzt und intensiv geschüttelt. Nach einer Standzeit von 70 Stunden wurde diese Mischung in ein beheizbares Doppelmantelrohr gefüllt, mit 100 l/h N₂ gespült und dabei langsam bis auf 210°C erwärmt. Das Erwärmen wurde so lange fortgesetzt, bis die dabei einsetzende H₂O-Abspaltung beendet war. Hierbei wurde mehr abgespaltenes Wasser beobachtet, als dem Verdünnungswasser der H₃PO₄ entsprach. Die dafür verantwortlichen Vorgänge sind noch nicht geklärt. Denkbar ist sowohl eine Kondensation der Phosphorsäure, etwa in Richtung zur Pyrophosphorsäure, als auch die Ausbildung von Bindungen zwischen der H₃PO₄/H₄P₂O₇ und OH-Gruppen auf der Oberfläche das SiO₂ .

### Beispiel 1

In einer kontinuierlichen Apparatur aus Einsatzbehälter, Pumpe, elektrischem Ofen mit eingesetztem Quarzglasrohr (Länge in der Ofenzone 50 cm, Durchmesser 3 cm), Abscheider mit Trockeneis-Kühlfalle und Gasuhr wurde Propylenoxid von oben nach unten mit einer LHSV von 0,2 über ein SiO₂-Festbett (SiO₂ von Fa. Grace) geleitet. Die Temperatur am Katalysatorbett wurde auf 280°C eingestellt; der Propylenoxid-Gasstrom wurde mit der 1,5 fachen molaren Menge N₂ verdünnt. Das nach dem Reaktor auskondensierte Reaktionsprodukt wurde gaschromatographisch untersucht. Im Vergleich zu diesem nicht mit H₃PO₄ dotierten SiO₂ wurde in einem weiteren Versuchslauf der Katalysator nach der oben beschriebenen Herstellungsweise eingesetzt. Die Katalysatoren und die Versuchsergebnisse sind in Tabelle 1 aufgeführt.

Es zeigte sich, daß durch die Belegung mit H₃PO₄ die Selektivität des Katalysators von 69 % auf über 83 % gesteigert werden kann.

**Tabelle 1**

| Isomerisierung von Propylenoxid zu Propanal | | |
|---|---|---|
| Reaktionsprodukt/Katalysator | SiO₂ | SiO₂ mit H₃PO₄ |
| Propox (Gew.-%) | 3,81 | 1,5 |
| Propanal (Gew.-%) | 60,65 | 82,1 |
| Aceton (Gew.-%) | 6,41 | 7,33 |
| Allylalkohol (Gew.-%) | 1,79 | 1,51 |
| Sonst. (Gew.-%) | 27,34 | 7,57 |
| Umsatz (%) | 96,19 | 98,5 |
| Ausbeute (%) | 60,65 | 82,1 |
| Selektivität (%) | 63,05 | 83,35 |

### Beispiel 2 und 3

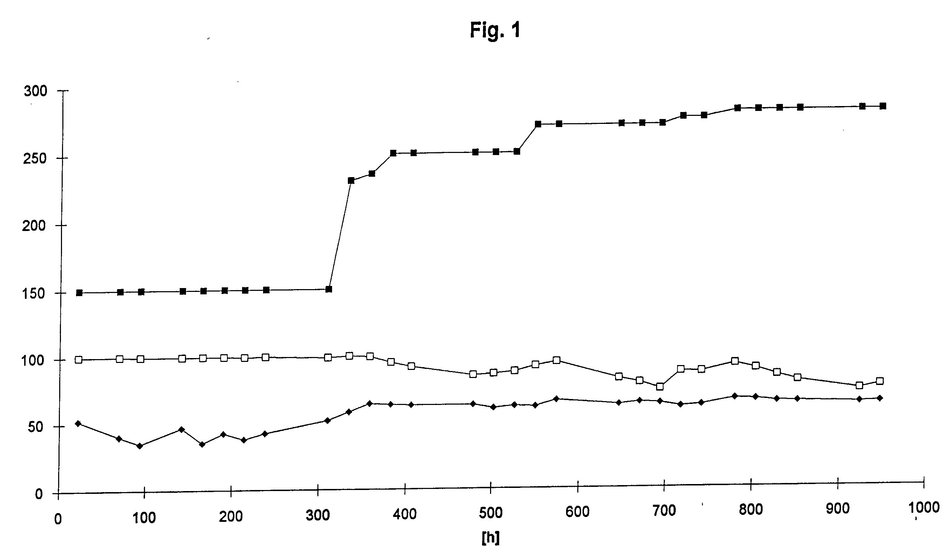

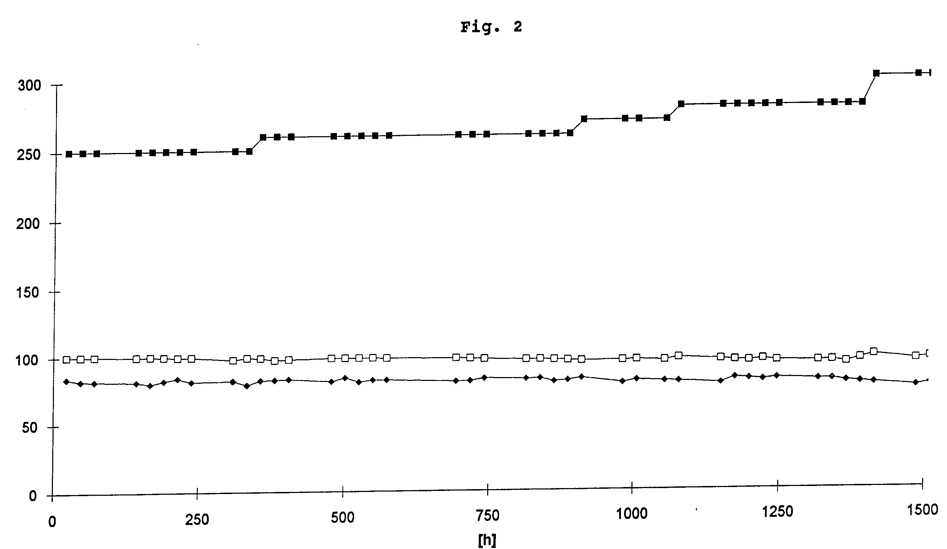

In einer Apparatur wie in Beispiel 1 wurde das Langzeitverhalten eines H₃PO₄/SiO₂-Katalysators und eines sauren Schichtsilikats (Hersteller: u.a. Fa. Engelhard) untersucht. Fig. 1 zeigt die von 150°C langsam auf 300°C gesteigerte Reaktionstemperatur (auf der Kante liegende schwarze Karos), die dazu gehörenden Umsatzwerte in Prozent (auf der Kante liegende weiße Karos) und die dabei erhaltenen Selektivitäten in Prozent (auf der Spitze stehende schwarze Karos) für das eingesetzte Schichtsilikat als Katalysator (Beispiel 2). Man erkennt aus Fig. 1, daß die Temperatursteigerung nicht vollständig in der Lage war, den langsam abfallenden Umsatz nahe bei 100 % zu halten. Fig. 2 zeigt den Langzeitversuch des H₃PO₄/SiO₂-Katalysators (Beispiel 3), bei dem die Temperatur langsam von 250 auf 300°C angehoben wurde (auf der Kante stehende schwarze Karos), wodurch der Umsatz (auf der Kante stehende weiße Karos) sehr konstant blieb und in der Nähe von 100 % angesiedelt war und wozu eine sehr konstante Selektivität (auf der Spitze stehende schwarze Karos) im Bereich von etwa 80 % gehörte. Die Versuche wurden nach jeweils über 1500 Stunden abgebrochen. Auf der Abszisse der jeweiligen Figur ist die Laufzeit des Versuches in Stunden aufgetragen; auf der Ordinate einer jeden Figur sind die Zahlenwerte der Temperatur in °C, sowie des Umsatzes und der Selektivität in % aufgetragen. Bei den Langzeitversuchen wurde deutlich, daß der H₃PO₄-beladene SiO₂-Katalysator eine höhere Selektivität zeigte als das Schichtsilikat. Jedoch bewahrte das Schichtsilitkat im Langzeitversuch eine hohe Standfestigkeit, während das im Beispiel 1 benutzte SiO₂ ohne H₃PO₄-Beladung, also ohne saure Eigenschaften, schon nach 2 Tagen einen schaffen Abfall der katalytischen Wirksamkeit zeigte.

## Patentansprüche

1. Verfahren zur Herstellung von Propanal, dadurch gekennzeichnet, daß Propylenoxid bei 150 bis 400°C an einem sauren SiO₂-haltigen Material in der Gasphase isomerisiert wird.

2. Verfahren nach Anspruch 1, dadurch gekennzeichnet, daß die Temperatur 170 bis 320°C beträgt.

3. Verfahren nach Anspruch 1, dadurch gekennzeichnet, daß bei einer Strömungsgeschwindigkeit LHSV von 0,01-2 h⁻¹, bevorzugt 0,1-1 h⁻¹ gearbeitet wird.

4. Verfahren nach Anspruch 1, dadurch gekennzeichnet, daß Propylenoxid (PO) mit Inertgas im Volumenverhältnis PO:Inertgas = 1:0,1 bis 5, bevorzugt 1:1-3 eingesetzt wird.

5. Verfahren nach Anspruch 1, dadurch gekennzeichnet, daß ein Teil des abströmenden Reaktionsgemisches einer destillativen Aufarbeitung zur Gewinnung des Propanals zugeführt wird und der Rest gemeinsam mit frischem Propylenoxid erneut in die katalytische Isomerisierung zurückgeführt wird.

6. Verfahren nach Anspruch 1, dadurch gekennzeichnet, daß als saures SiO₂-haltiges Material ein H₃PO₄-beladenes SiO₂, bevorzugt ein H₃PO₄-beladenes SiO₂-Gel, eingesetzt wird.

7. Verfahren nach Anspruch 6, dadurch gekennzeichnet, daß der H₃PO₄-Gehalt 10-65 %, bevorzugt 15-45 % des Gesamtgewichts von H₃PO₄ und SiO₂ ausmacht.
